# DEMANDE DE BREVET EUROPEEN

(11) **EP 0 583 198 A1**
(43) Date de publication de la demande: **16.02.1994**
(21) Numéro de dépôt: 93402033.0
(22) Date de dépôt: 10.08.1993
(51) Int. Cl.: C07D 401/06, C07D 233/52, A01N 43/90, A01N 43/50

(54) **Dérivés de l'imidazole, leur procédé de préparation et leur procédé de préparation et leur application comme pesticides**

(30) Priorité: 11.08.1992 FR 9209905
(71) Demandeur: ROUSSEL-UCLAF, F-75007 Paris (FR)
(72) Inventeur: Benoit, Marc, F-13360 Roquevaire (FR); Demoute, Jean-Pierre, F-93360 Neuilly Plaisance (FR); Pastre, Pierre, F-13008 Marseille Cedex 08 (FR); Ugolini, Antonio, F-91300 Massy (FR)
(74) Mandataire: Niel, Michel

(57) **Abrégé**

L'invention a pour objet les composés de formule (I) : 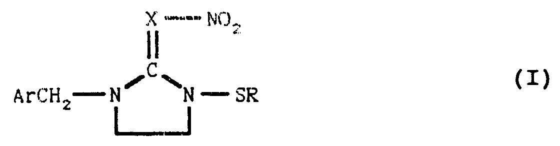
dans laquelle :
- Ar représente un radical aryle ou hétéroaryle éventuellement substitué,
- X représente un atome d'azote ou un groupement =CH-,
- R représente un radical alkyle, alkényle ou alkynyle, éventuellement substitué, ou un radical Salc, alc représentant un radical alkyle éventuellement substitué.

Les composés de l'invention présentent des propriétés pesticides.

## Description

La présente invention concerne de nouveaux dérivés de l'imidazolidine, leur procédé de préparation et leur application comme pesticides.

L'invention a pour objet les composés de formule (I) : 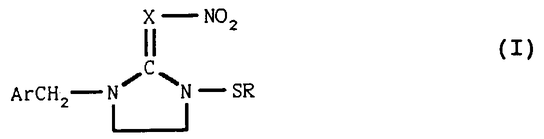
dans laquelle :
- Ar représente un radical aryle ou hétéroaryle éventuellement substitué par un ou plusieurs substituants choisis dans le groupe constitué par les atomes d'halogène, les radicaux alkyle renfermant jusqu'à 4 atomes de carbone, linéaires, ramifiés ou cycliques éventuellement substitués par un ou plusieurs atomes d'halogène, les radicaux SH et OH libres, estérifiés et éthérifiés,
- X représente un atome d'azote ou un groupement =CH-,
- R représente un radical alkyle, alkényle ou alkynyle, linéaire, ramifié ou cyclique renfermant jusqu'à 12 atomes de carbone éventuellement substitué par un ou plusieurs atomes d'halogène, ou un radical Salc, alc représentant un radical alkyle renfermant jusqu'à 4 atomes de carbone éventuellement substitué par un ou plusieurs atomes d'halogène.

Comme radical aryle, on peut citer le radical phényle ou naphtyle.

Comme radical hétéroaryle, on peut citer le radical pipéridyle, le radical pyrimidyle, le radical pyridyle, le radical thiazolyle, le radical oxazolyle et le radical morpholinyle.

Lorsque Ar est substitué par un ou plusieurs atomes d'halogène, l'halogène est de préférence le chlore ou le brome.

Lorsque Ar est substitué par un ou plusieurs radicaux alkyle, il s'agit de préférence du radical méthyle, éthyle ou n-propyle.

Lorsque Ar est substitué par un radical OH ou SH estérifié, le reste ester est de préférence du radical acétyle, propionyle ou benzoyle.

Lorsque Ar est substitué par un radical OH ou SH éthérifié, le reste éther de préférence du radical méthyle ou éthyle.

Lorsque R représente un radical alkyle, il s'agit de préférence d'un radical méthyle, éthyle, propyle, isopropyle, n-butyle, isobutyle, terbutyle, n-pentyle ou n-hexyle, d'un radical cyclopropyle, cyclobutyle, cyclopentyle ou cyclohexyle.

Lorsque R représente un radical Salc, alc représente de préférence un radical S-méthyle, S-éthyle, S-propyle, S-butyle, S-isobutyle ou S-terbutyle.

Lorsque R est substitué par un ou plusieurs atomes d'halogène, l'halogène est de préférence le chlore ou le brome.

Parmi les composés préférés de l'invention, on peut citer les composés dans lesquels Ar représente un radical pyridinyle éventuellement substitué, par exemple les composés dans lesquels Ar représente un radical 6-chloro 3-pyridinyle, on peut citer également les composés de formule (I) dans lesquels X représente un atome d'azote.

L'invention a notamment pour objet les composés de formule (I) dans lesquels R représente un radical alkyle, linéaire, ramifié ou cyclique renfermant jusqu'à 8 atomes de carbone éventuellement substitué par un ou plusieurs atomes d'halogène, comme par exemple un radical terbutyle, ou encore un radical trichlorométhyle.

L'invention a tout spécialement pour objet, les composés des exemples 3 et 5A.

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 5 g et 300 g de matière active à l'hectare.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment le produit de l'exemple 3 ou de l'exemple 5A.

L'invention a notamment pour objet les compositions insecticides renfermant comme principe actif au moins l'un des produits définis ci-dessus.

L'invention a également pour objet les compositions acaricides renfermant comme principe actif au moins l'un des produits définis ci-dessus.

L'invention a encore pour objet les compositions nématicides renfermant comme principe actif au moins l'un des produits définis ci-dessus.

L'invention a enfin pour objet les compositions acaricides destinées à la lutte contre les parasites des animaux à sang chaud, notamment contre les tiques et les gales, renfermant comme principe actif, au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de matière active ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]hept-5-ène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet un procédé de préparation des composés de formule (I), tels que définis à l'une quelconque des revendications 1 à 8, caractérisé en ce que l'on soumet un composé de formule (II) : 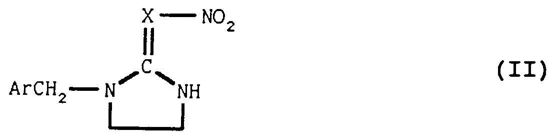
dans laquelle Ar et X sont définis comme précédemment, à l'action d'un composé de formule (III) :

RSHal

dans laquelle R est défini comme précédemment, en présence d'une base pour obtenir le composé de formule (I) correspondant.

Dans un mode de réalisation préféré du procédé de l'invention :
- le composé de formule (III) est préparé "in situ", par action du chlorure de sulfuryle sur un disulfure de formule RSSR, ou encore par action du chlore sur un disulfure de formule RSSR,
- la base utilisée est de préférence l'hydrure de sodium, un alcoolate alcalin ou alcalinoterreux, un amidure alcalin, une amine secondaire ou tertiaire en présence de bromure de lithium.

Les produits de formule (II) utilisés comme produits de départ sont décrits dans le brevet européen 192060.

Les exemples suivants illustrent l'invention sans toutefois la limiter.

### EXEMPLE 1 : 1-[(6-chloro 3-pyridinyl) méthyl] N-nitro 3-[(méthyléthyl) thio] 2-imidazolidinimine,

### a) Préparation du chlorure de sulfure de méthyléthyle

On introduit à -20°C, 5,25 cm³ de chlorure de sulfuryle dans 10,3 cm³ de disulfure de méthyléthyle. On réchauffe le mélange réactionnel à 0°C et l'agite à cette température pendant 1 h 30.

On enlève le dioxyde de soufre sous pression réduite progressive et distille sous 43 millibars. On obtient 11 g de produit recherché. Eb _{43mb} = 28°C.

### b) Condensation

On introduit 2,55 g de 1-[(6-chloro 3-pyridinyl) méthyl] N-nitro 2-imidazolidinimine dans 40 cm³ de tétrahydrofuranne. On ajoute 0,48 g d'hydrure de sodium à 50 % dans l'huile. On agite pendant 30 minutes et refroidit vers -50°C. On introduit ensuite 6 cm³ d'une solution renfermant 11 g de produit préparé ci-dessus au paragraphe a), dans 50 cm³ de dichlorométhane. On maintient le mélange réactionnel sous agitation pendant 1 heure en laissant la température remonter vers -20°C. On verse dans un bain d'eau et de glace. On extrait au chlorure de méthylène, sèche et amène à sec, sous pression réduite. On obtient un produit que l'on chromatographie sur silice, en éluant avec le mélange chlorure de i méthylène-méthanol (95-5). On obtient ainsi 1,75 g du produit recherché, fondant après recristallisation dans l'acétate d'éthyle à 110°C.

### EXEMPLE 2 : 1-[(6-chloro 3-pyridinyl) méthyl] N-nitro 3-[(cyclohexyl) thio] 2-imidazolidinimine

### a) Préparation du chlorure de sulfure de cyclohexyle

On introduit 0,77 g de disulfure de dicyclohexyle dans 5 cm³ de tétrachlorure de carbone. On refroidit à -10°C et fait barboter un courant de chlore jusqu'à fixer 0,24 g de chlore. On agite pendant 10 minutes à -10°C. On obtient ainsi une solution que l'on utilise telle quelle dans le stade suivant.

### b) Condensation

On introduit 1,27 g de 1-[(6-chloro 3-pyridinyl) méthyl] N-nitro 2-imidazolidinimine dans 20 cm³ de tétrahydrofuranne. On introduit ensuite 0,29 g d'hydrure de sodium à 50 % en solution dans l'huile. On agite pendant 30 minutes, refroidit à -20°C et introduit en 10 minutes la solution préparée au paragraphe a). On agite pendant 10 minutes à -20°C et verse dans 50 cm³ d'une solution aqueuse saturée de carbonate acide de sodium. On agite et extrait au chlorure de méthylène. On décante, sèche et amène à sec. On obtient 2,9 g de produit que l'on chromatographie sur silice, en éluant avec le mélange chlorure de méthylène-méthanol (95-5). On recueille le produit de Rf = 0,55. On amène à sec. On obtient un produit que l'on recristallise dans l'acétate d'éthyle. On obtient 1,16 g de produit recherché fondant à 128°C.

### EXEMPLES 3 à 5 : en opérant comme précédemment, on a obtenu les produits suivants :

### Exemple 3 : 1-[(6-chloro 3-pyridinyl) méthyl) N-nitro 3-[(trichlorométhyl) thio] 2-imidazolidinimine

F = 137°C

### Exemple 4 : 1-[(6-chloro 3-pyridinyl) méthyl) N-nitro 3-(1-méthyl) propylthio] 2-imidazolidinimine

F = 116°C

### Exemple 5 : 1-[(6-chloro 3-pyridinyl) méthyl] 3-[(1,1-diméthyléthyl) dithio] N-nitro 2-imidazolidinimine (produit B) et 1-[(6-chloro 3-pyridinyl) méthyl] 3-[(1,1-diméthyl éthyl) thio] N-nitro 2-imidazolidinimine (produit A).

### a) Préparation du chlorure de sulfure de 1,1-diméthyléthyle.

On introduit 0,59 g de disulfure de diterbutyle dans 5 cm³ de tétrachlorure de carbone. On refroidit à -10°C et fait barboter un courant de chlore jusqu'à fixer 0,24 g de chlore. On agite ensuite pendant 10 minutes à -10°C. On obtient une solution que l'on utilise telle quelle.

### b) Condensation.

On introduit 1,27 g 1-[(6-chloro 3-pyridinyl) méthyl] N-nitro 2-imidazolidinimine dans 20 cm³ de tétrahydrofuranne. On introduit ensuite 0,29 g d'hydrure de sodium à 50 % dans l'huile. On agite pendant 30 minutes la solution obtenue, refroidit à -20°C et introduit la solution de chlorure de sulfure de 1,1-diméthyléthyle obtenue au stade a). On agite pendant 20 minutes à -20°C et verse dans une solution aqueuse saturée en carbonate acide de sodium. On agite, extrait au chlorure de méthyle, décante, sèche et amène à sec. On obtient 1,8 g de produit que l'on chromatographie sur silice en éluant avec le mélange chlorure de méthylène-méthanol (95-5). On obtient 0,7 g de produit B, Rf = 0,60, F = 134°C et 0,8 g de produit de Rf = 0,35, que l'on chromatographie sur silice en éluant avec le mélange chlorure de méthylène-acétate d'éthyle (7-3). On isole le produit de Rf = 0,25. On obtient 180 mg de produit A. F = 141°C.

### Etude de l'activité des composés de l'invention

### a) Etude de l'activité sur Aphis craccivora

Des plants de fèves sont traités par trempage des feuilles dans une solution hydroacétonique de matière active (50 % acétone, 50 % eau) puis séchées sous hotte aspirante.

Les feuilles sont ensuite infestées : 20 femelles d'Aphis craccivora adultes par feuille et maintenues à 22°C sous plafond lumineux.

Les contrôles de mortalité sont effectués au bout de 48 heures.

### Résultats : Dès la dose de 20 ppm, les produits présentent une activité aphicide intéressante.

### b) Etude de l'effet sur larves de Spodoptera littoralis par contact et ingestion.

On utilise des larves du stade L3 de Spodoptera littoralis. On opère à 22°C dans des conditions d'humidité relative de 50 %. On utilise des boîtes de PETRI renfermant un rond de papier filtre humidifié, dans chaque boîte on place deux feuilles de haricot traitées par une solution hydroacétonique (50-50) renfermant le produit à tester.

On fait le comptage des larves mortes au bout de 7 jours.

### c) Etude de l'effet sur Phaedon cochleariae.

On opère à 22°C dans des conditions d'humidité relative de 50 %. On utilise des boîtes de PETRI renfermant un rond de papier filtre humidifié et deux disques de feuille de chou chinois traités par une solution hydroacétonique (50-50) renfermant le produit à tester.

On fait le comptage des insectes morts au bout d'une semaine.

Les produits de l'invention ont une activité intéressante sur Spodoptera littoralis et Phaedon cochleariae.

## Revendications

**1)** Les composés de formule (I) : 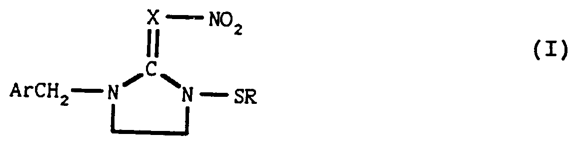 dans laquelle :
- Ar représente un radical aryle ou hétéroaryle éventuellement substitué par un ou plusieurs substituants choisis dans le groupe constitué par les atomes d'halogène, les radicaux alkyle renfermant jusqu'à 4 atomes de carbone, linéaires, ramifiés ou cycliques éventuellement substitués par un ou plusieurs atomes d'halogène, les radicaux SH et OH libres, estérifiés et éthérifiés,
- X représente un atome d'azote ou un groupement =CH-,
- R représente un radical alkyle, alkényle ou alkynyle, linéaire, ramifié ou cyclique renfermant jusqu'à 12 atomes de carbone éventuellement substitué par un ou plusieurs atomes d'halogène, ou un radical Salc, alc représentant un radical alkyle renfermant jusqu'à 4 atomes de carbone éventuellement substitué par un ou plusieurs atomes d'halogène.

**2)** Les composés de formule (I) tels que définis à la revendication 1, dans lesquels Ar représente un radical pyridinyle éventuellement substitué.

**3)** Les composés de formule (I) tels que définis à la revendication 2, dans lesquels Ar représente un radical 6-chloro 3-pyridinyle.

**4)** Les composés de formule (I) tels que définis à l'une quelconque des revendications 1 à 3 dans lesquels X représente un atome d'azote.

**5)** Les composés de formule (I) tels que définis à l'une quelconque des revendications 1 à 4 dans lesquels R représente un radical alkyle, linéaire, ramifié ou cyclique renfermant jusqu'à 8 atomes de carbone éventuellement substitué par un ou plusieurs atomes d'halogène.

**6)** Les composés de formule (I) tels que définis à la revendication 5 dans lesquels R représente un radical terbutyle.

**7)** Les composés de formule (I) tels que définis à la revendication 5 dans lesquels R représente un radical trichlorométhyle.

**8)** Les composés de formule (I) tels que définis à la revendication 1 dont les noms suivent :
- la 1-[(6-chloro 3-pyridinyl) méthyl] N-nitro 3-[(trichlorométhyl) thio] 2-imidazolidinimine,
- la 1-[(6-chloro 3-pyridinyl) méthyl] N-nitro 3-[(1,1-diméthyléthyl) thio] 2-imidazolidinimine.

**9)** Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 7.

**10)** Les compositions destinées à la lutte contre les parasites des végétaux, les parasites des locaux et les parasites des animaux à sang chaud, caractérisées en ce qu'elles renferment comme principe actif au moins un des produits définis à la revendication 8.

**11)** Les compositions insecticides renfermant comme principe actif au moins l'un des produits définis à l'une quelconque des revendications 1 à 8.

**12)** Les compositions acaricides renfermant comme principe actif au moins l'un des produits définis à l'une quelconque des revendications 1 à 8.

**13)** Les compositions nématicides renfermant comme principe actif au moins l'un des produits définis à l'une quelconque des revendications 1 à 8.

**14)** Les compositions acaricides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 8.

**15)** Procédé de préparation des composés de formule (I), tels que définis à l'une quelconque des revendications 1 à 8, caractérisé en ce que l'on soumet un composé de formule (II) : 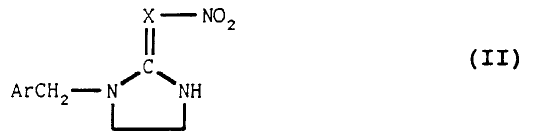 dans laquelle Ar et X sont définis comme précédemment, à l'action d'un composé de formule (III) :
RSHal
dans laquelle R est défini comme précédemment, en présence d'une base pour obtenir le composé de formule (I) correspondant.
